(19) Europäisches Patentamt
European Patent Office
Office européen des brevets

(11) EP 4 354 578 A1

(12) EUROPEAN PATENT APPLICATION

(43) Date of publication:
17.04.2024 Bulletin 2024/16

(21) Application number: 23195155.9

(22) Date of filing: 04.09.2023

(51) International Patent Classification (IPC):
*H01M 10/0567 (2010.01)* *H01M 10/0569 (2010.01)*
*H01M 10/0525 (2010.01)* *H01M 4/525 (2010.01)*
*H01M 4/36 (2006.01)* *H01M 4/38 (2006.01)*
*H01M 4/587 (2010.01)*

(52) Cooperative Patent Classification (CPC):
**H01M 10/0567; H01M 4/525; H01M 10/0525; H01M 10/0569;** H01M 4/364; H01M 4/386; H01M 4/587; H01M 2300/0037

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 13.10.2022 KR 20220131830

(71) Applicant: **Samsung SDI Co., Ltd.**
**Yongin-si, Gyeonggi-do 17084 (KR)**

(72) Inventors:
- **Yang, Yeji**
**17084 Yongin-si, Gyeonggi-do (KR)**
- **Choi, Hyunbong**
**17084 Yongin-si, Gyeonggi-do (KR)**
- **Kim, Sundae**
**17084 Yongin-si, Gyeonggi-do (KR)**
- **Park, Sangwoo**
**17084 Yongin-si, Gyeonggi-do (KR)**
- **Kim, Dahyun**
**17084 Yongin-si, Gyeonggi-do (KR)**
- **Park, Hongryeol**
**17084 Yongin-si, Gyeonggi-do (KR)**
- **Kim, Sanghoon**
**17084 Yongin-si, Gyeonggi-do (KR)**

(74) Representative: **Gulde & Partner**
**Patent- und Rechtsanwaltskanzlei mbB**
**Wallstraße 58/59**
**10179 Berlin (DE)**

# (54) RECHARGEABLE LITHIUM BATTERY

(57) Disclosed are a rechargeable lithium battery including an electrolyte solution including a non-aqueous organic solvent, a lithium salt, and an additive; a positive electrode including a positive electrode active material; and a negative electrode including a negative electrode active material, wherein the non-aqueous organic solvent includes ethylene carbonate in an amount of less than about 5 wt%, and the additive includes a compound represented by Chemical Formula 1, and
the positive electrode active material is represented by Chemical Formula 2.

Chemical Formula 1 and Chemical Formula 2 are the same as defined in the specification.

EP 4 354 578 A1

## Description

## BACKGROUND OF THE INVENTION

### (a) Field of the Invention

This disclosure relates to a rechargeable lithium battery.

### (b) Description of the Related Art

A rechargeable lithium battery may be recharged and has three or more times as high energy density per unit weight as a conventional lead storage battery, nickel-cadmium battery, nickel hydrogen battery, nickel zinc battery and the like. It may be also charged at a high rate and thus, is commercially manufactured for a laptop, a cell phone, an electric tool, an electric bike, and the like, and researches on improvement of additional energy density have been actively made.

Particularly, as IT devices are increasingly achieving high-performance, high-capacity batteries are required, and the high capacity may be realized through expansion of a voltage region, which may increase energy density, but in the high-voltage region, there is a problem of deteriorating performance of a positive electrode due to oxidization of an electrolyte solution.

In particular, since a cobalt-free lithium nickel manganese-based oxide as a positive electrode active material includes not cobalt but nickel, manganese, and the like as a main component in a positive electrode active material composition, a positive electrode including this cobalt-free lithium nickel manganese-based oxide as a positive electrode active material is economical and realizes high energy density and thus draws lots of attentions as a next generation positive electrode active material.

However, when the positive electrode including the cobalt-free lithium nickel manganese-based oxide is used in a high-voltage environment, transition metals are eluted therefrom due to structural collapse of the positive electrode, which may cause a problem such as gas generation inside a cell, capacity reduction, and the like. This transition metals tends to be more eluted in a high-temperature environment and precipitated on the surface of a negative electrode and thus cause a side reaction, which leads to an increase in battery resistance, deterioration of cycle-life and output characteristics of a battery.

Accordingly, when the positive electrode including the cobalt-free lithium nickel manganese-based oxide is used, an electrolyte solution applicable under high-voltage and high-temperature conditions is required.

## SUMMARY OF THE INVENTION

An embodiment provides a rechargeable lithium battery exhibiting improved high-voltage characteristics and high-temperature characteristics by combining a layered positive electrode active material including a cobalt-free lithium nickel manganese-based oxide and an electrolyte solution effectively protecting a positive electrode including the positive electrode active material to reduce transition metal elution under high-voltage and high-temperature conditions and suppress structural collapse of the positive electrode.

An embodiment provides a rechargeable lithium battery including an electrolyte solution including a non-aqueous organic solvent, a lithium salt, and an additive; a positive electrode including a positive electrode active material; and a negative electrode including a negative electrode active material,

wherein the non-aqueous organic solvent includes ethylene carbonate in an amount of less than 5 wt%,
the additive includes a compound represented by Chemical Formula 1, and the positive electrode active material is represented by Chemical Formula 2.

[Chemical Formula 1]

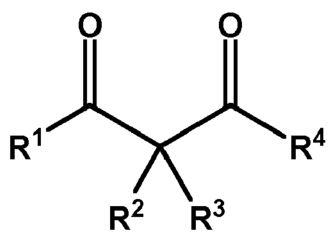

In Chemical Formula 1,

$R^1$ to $R^4$ are each independently hydrogen, a halogen, a cyano group, a substituted or unsubstituted C1 to C20 alkyl group, a substituted or unsubstituted C6 to C20 aryl group, or a substituted or unsubstituted C2 to C30 heterocyclic group;

[Chemical Formula 2] $Li_aNi_xMn_{1-x-y}A_yO_{2\pm b}X_c$

wherein, in Chemical Formula 2,

$$0.9 \leq a \leq 1.2,\ 0 \leq b \leq 0.1,\ 0 \leq c \leq 0.1,\ 0.5 \leq x \leq 0.95, \text{ and } 0 \leq y < 0.3,$$

A is at least one element selected from B, Na, Mg, Al, Ti, and Si, and
X is at least one element selected from S, F, P, and Cl.

The non-aqueous organic solvent may be composed of only chain carbonate.
The chain carbonate may be represented by Chemical Formula 3.

[Chemical Formula 3]

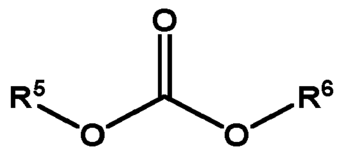

In Chemical Formula 3
$R^5$ and $R^6$ are each independently a substituted or unsubstituted C1 to C20 alkyl group.
The non-aqueous organic solvent may be a mixed solvent comprising at least two of dimethyl carbonate (DMC), diethyl carbonate (DEC), dipropyl carbonate (DPC), methylpropyl carbonate (MPC), ethylpropyl carbonate (EPC), and ethylmethyl carbonate (EMC).
The non-aqueous organic solvent may include ethylmethyl carbonate (EMC) and dimethyl carbonate (DMC) in a weight ratio of about 0 : 100 to about 50 : 50.
At least one selected from $R^1$ to $R^4$ in Chemical Formula 1 may be an unsubstituted C1 to C20 alkyl group, a C1 to C20 alkyl group substituted with an electron withdrawing group, an unsubstituted C6 to C20 aryl group, a C6 to C20 aryl group substituted with an electron withdrawing group, an unsubstituted C2 to C30 heterocyclic group, or a C2 to C30 heterocyclic group substituted with an electron withdrawing group.
The electron withdrawing group may be at least one selected from a halogen, an isocyanate group (-NCO), an isothiocyanate group (-NCS), a cyanate group (-OCN), a thiocyanate group (-SCN), a cyano group (-CN), an isocyano group (-NC), a -N=C=N-group, a -N=S=N- group, a nitro group ($NO_2$), a trifluoromethane ($CF_3$) group, a pentafluoroethane ($C_2F_5$) group, a trifluoromethane sulfonyl ($SO_2CF_3$) group, a pentafluoroethane sulfonyl ($SO_2C_2F_5$) group, a trifluoromethane sulfonate ($SO_3CF_3$) group, a pentafluoroethane sulfonate ($SO_3C_2F_5$) group, a pentafluorophenyl ($C_6F_5$) group, an acetyl ($COCH_3$) group, an ethyl ketone ($COC_2H_5$) group, a propyl ketone ($COC_3H_7$) group, a butyl ketone ($COC_4H_9$) group, a pentyl ketone ($COC_5H_{11}$) group, a hexyl ketone ($COC_6H_{13}$) group, an ethanoate ($CO_2CH_3$) group, a propanoate ($CO_2C_2H_5$) group, a butaneoate ($CO_2C_3H_7$) group, a pentanoate ($CO_2C_4H_9$) group, and a hexanoate ($CO_2C_5H_{11}$) group.
At least one of $R^2$ and $R^3$ in Chemical Formula 1 may be hydrogen.
$R^2$ and $R^3$ of Chemical Formula 1 may each be hydrogen.
The compound represented by Chemical Formula 1 may be selected from compounds of Group 1.

[Group 1]

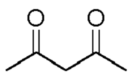 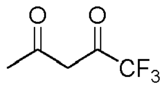 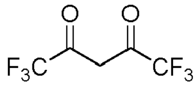 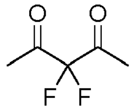 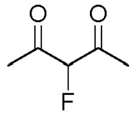

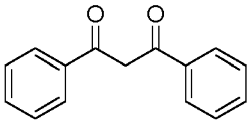 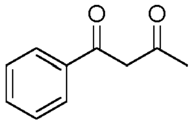 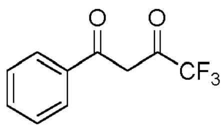 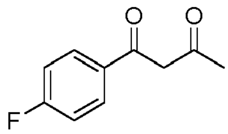

The compound represented by Chemical Formula 1 may be included in an amount of about 0.01 to about 5.0 parts by weight based on 100 parts by weight of the electrolyte solution for a rechargeable lithium battery.

The electrolyte solution may further include at least one other additives selected from vinylene carbonate (VC), fluoroethylene carbonate (FEC), difluoroethylene carbonate, chloroethylene carbonate, dichloroethylene carbonate, bromoethylene carbonate, dibromoethylene carbonate, nitroethylene carbonate, cyanoethylene Carbonate, vinylethylene carbonate (VEC), adiponitrile (AN), succinonitrile (SN), 1,3,6-hexane tricyanide (HTCN), propensultone (PST), propanesultone (PS), lithium tetrafluoroborate ($LiBF_4$), lithium difluorophosphate ($LiPO_2F_2$), and 2-fluorobiphenyl (2-FBP).

In Chemical Formula 2, x may be $0.8 \leq x \leq 0.95$.

The positive electrode active material may include $LiNi_{0.75}Mn_{0.25}O_2$, $LiNi_{0.80}Mn_{0.20}O_2$, $LiNi_{0.85}Mn_{0.15}O_2$, $LiNi_{0.90}Mn_{0.10}O_2$, $LiNi_{0.95}Mn_{0.05}O_2$, or a solid solution thereof.

The negative electrode active material may include at least one of graphite and a Si composite.

The rechargeable lithium battery may have a charging upper limit voltage of greater than or equal to about 4.35 V.

An embodiment uses a combination of a layered positive electrode active material including a cobalt-free lithium nickel manganese-based oxide and an electrolyte solution effectively protecting a positive electrode including the layered positive electrode active material to secure phase transition safety of the positive electrode even in an high-temperature and high-voltage environment and to reduce gas generation by suppressing decomposition of the electrolyte solution and a side reaction with electrodes and simultaneously, to improve battery stability and cycle-life characteristics of a rechargeable lithium battery by suppressing an increase in internal battery resistance.

## BRIEF DESCRIPTION OF THE DRAWINGS

FIG. 1 is a schematic view showing a rechargeable lithium battery according to an embodiment.

## DETAILED DESCRIPTION OF THE EMBODIMENTS

Hereinafter, a rechargeable lithium battery according to an embodiment of the present disclosure will be described in detail with reference to the accompanying drawings. However, these embodiments are exemplary, the present invention is not limited thereto and the present invention is defined by the scope of claims.

As used herein, when a definition is not otherwise provided "substituted" refers to replacement of at least one hydrogen of a substituent or a compound by deuterium, a halogen, a hydroxyl group, an amino group, a C1 to C30 amine group, a nitro group, a C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 trifluoroalkyl group, a C1 to C10 fluoroalkyl group, an isocyanate group (-NCO), an isothiocyanate group (-NCS), a cyanate group (-OCN), a thiocyanate group (-SCN), a cyano group (-CN), an isocyano group (-NC) or a combination thereof.

In one example of the present invention, "substituted" refers to replacement of at least one hydrogen of a substituent or a compound by deuterium, a halogen, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C10 fluoroalkyl group, a C1 to C10 trifluoroalkyl group, or a cyano group. In addition, in specific examples of the present invention, "substituted" refers to replacement of at least one hydrogen of a substituent or a compound by deuterium, a halogen, a C1 to C20 alkyl group, a C6 to C30 aryl group, a C1 to C10 fluoroalkyl group, a C1 to C10 trifluoroalkyl group, or a cyano group. In addition, in specific examples of the present invention, "substituted" refers to replacement of at least one hydrogen of a substituent or a compound by deuterium, a halogen, a C1 to C5 alkyl group, a C6 to C18 aryl group, a C1 to C5 fluoroalkyl group, a C1 to C5 trifluoroalkyl group, or a cyano group. In addition, in specific examples of the present invention, "substituted" refers to replacement of at least one hydrogen of a substituent

or a compound by deuterium, a cyano group, a halogen, a methyl group, an ethyl group, a propyl group, a butyl group, a phenyl group, a biphenyl group, a terphenyl group, trifluoromethyl group, or a naphthyl group.

A rechargeable lithium battery may be classified into a lithium ion battery, a lithium ion polymer battery, and a lithium polymer battery depending on kinds of a separator and an electrolyte solution. It also may be classified to be cylindrical, prismatic, coin-type, pouch-type, and the like depending on shape. In addition, it may be bulk type or thin film type depending on sizes. Structures and manufacturing methods for lithium ion batteries pertaining to this disclosure are well known in the art.

Herein, as an example of a rechargeable lithium battery, a cylindrical rechargeable lithium battery is for example described. FIG. 1 schematically shows the structure of a rechargeable lithium battery according to an embodiment. Referring to FIG. 1, a rechargeable lithium battery 100 according to an embodiment includes a battery cell including a positive electrode 114, a negative electrode 112 facing to the positive electrode 114, and a separator 113 between the positive electrode 114 and the negative electrode 112, and an electrolyte solution (not shown) impregnating the positive electrode 114, the negative electrode 112 and the separator 113, a battery case 120 housing the battery cell, and a sealing member 140 for sealing the batter case 120.

Hereinafter, a more detailed configuration of the rechargeable lithium battery 100 according to an embodiment is described.

The rechargeable lithium battery according to an embodiment of the present invention includes an electrolyte solution, a positive electrode, and a negative electrode.

The electrolyte solution includes a non-aqueous organic solvent, a lithium salt, and an additive, wherein the non-aqueous organic solvent includes ethylene carbonate in an amount of less than 5 wt%, and the additive includes a compound represented by Chemical Formula 1.

[Chemical Formula 1]

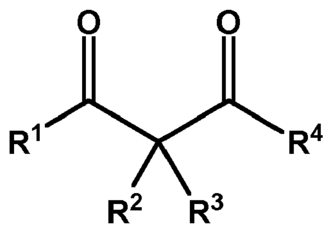

In Chemical Formula 1,

$R^1$ to $R^4$ are each independently hydrogen, a halogen, a cyano group, a substituted or unsubstituted C1 to C20 alkyl group, a substituted or unsubstituted C6 to C20 aryl group, or a substituted or unsubstituted C2 to C30 heterocyclic group.

The positive electrode includes a positive electrode active material including lithium nickel manganese-based oxide represented by Chemical Formula 2.

The lithium nickel manganese-based oxide represented by Chemical Formula 2 is a cobalt-free lithium nickel-manganese-based oxide and may be a layered positive electrode active material. In the case of a positive electrode active material including cobalt-free lithium nickel manganese-based oxide, solvent decomposition and elution of transition metals, particularly Ni, may occur due to strong structural instability under a high-voltage condition.

Due to such an elution phenomenon of transition metals, deterioration and short-circuiting may occur, resulting in a decrease in cycle-life capacity of the battery and a rapid increase in resistance.

However, in the case of using the aforementioned electrolyte solution together, it is possible to alleviate the decrease in the cycle-life capacity of the battery and the rapid increase in resistance.

In particular, by using the aforementioned positive electrode active material in an electrolyte solution containing less than 5 wt% of ethylene carbonate, specifically greater or equal to 0 wt% and less less than about 5 wt% of ethylene carbonate, the elution of transition metals may be effectively reduced under high voltage and high-temperature conditions, thereby suppressing collapse of the positive electrode structure and improving high-voltage characteristics and high-temperature characteristics.

The non-aqueous organic solvent serves as a medium for transmitting ions taking part in the electrochemical reaction of a battery.

The non-aqueous organic solvent may include a carbonate-based, ester-based, ether-based, ketone-based, alcohol-based, or aprotic solvent.

The carbonate-based solvent may include dimethyl carbonate (DMC), diethyl carbonate (DEC), dipropyl carbonate (DPC), methylpropyl carbonate (MPC), ethylpropyl carbonate (EPC), ethylmethyl carbonate (EMC), ethylene

carbonate (EC), propylene carbonate (PC), butylene carbonate (BC), and the like. The ester-based solvent may include methyl acetate, ethyl acetate, n-propyl acetate, t-butyl acetate, methyl propionate, ethyl propionate, propyl propionate, decanolide, mevalonolactone, caprolactone, and the like. The ether-based solvent may include dibutyl ether, tetraglyme, diglyme, dimethoxyethane, 2-methyltetrahydrofuran, tetrahydrofuran, and the like. In addition, the ketone-based solvent may include cyclohexanone, and the like. In addition, the alcohol-based solvent may include ethanol, isopropyl alcohol, and the like and the aprotic solvent may include nitriles such as R-CN (wherein R is C2 to C20 linear, branched, or cyclic hydrocarbon group and includes a double bonded, an aromatic ring or an ether bond), and the like, amides such as dimethyl formamide, dioxolanes such as 1,3-dioxolane, sulfolanes, and the like.

The non-aqueous organic solvents may be used alone or in combination with one or more of them, and when used in combination with one or more, the mixing ratio may be appropriately adjusted according to the desired battery performance, which is widely understood by those skilled in the art.

The non-aqueous organic solvent includes less than 5 wt% of ethylene carbonate.

When the content of ethylene carbonate is greater than or equal to 5 wt%, activity of Ni increases during high-voltage driving, the oxidation number of Ni tends to be reduced from tetravalent to divalent, and ethylene carbonate with low oxidation stability is oxidatively decomposed, resulting in Ni being eluted and deposited on the negative electrode.

As a specific example, the non-aqueous organic solvent may be composed of only chain carbonate. In this case, as the resistance increase rate during high-temperature storage is significantly alleviated, excellent high-temperature storage characteristics may be implemented.

In this specification, the meaning of composed of only chain carbonates means including organic solvents belonging to the category of chain carbonates alone or in combination without being mixed with cyclic carbonates and the like.

In an embodiment, the chain carbonate may be represented by Chemical Formula 3.

[Chemical Formula 3]

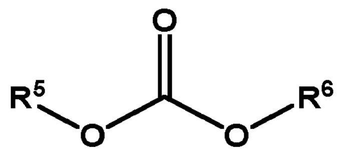

In Chemical Formula 3

$R^5$ and $R^6$ are each independently a substituted or unsubstituted C1 to C20 alkyl group.

For example, $R^5$ and $R^6$ in Chemical Formula 3 may each independently be a substituted or unsubstituted C1 to C10 alkyl group, and for example, $R^5$ and $R^6$ may each independently be a substituted or unsubstituted C1 to C5 alkyl group.

In an embodiment, $R^5$ and $R^6$ in Chemical Formula 3 may each independently be a substituted or unsubstituted methyl group, a substituted or unsubstituted ethyl group, a substituted or unsubstituted n-propyl group, a substituted or unsubstituted n-butyl group, a substituted or unsubstituted n-pentyl group, a substituted or unsubstituted iso-butyl group, or a substituted or unsubstituted neo-pentyl group.

For example, the non-aqueous organic solvent according to a specific embodiment may include at least two of dimethyl carbonate (DMC), diethyl carbonate (DEC), dipropyl carbonate (DPC), methylpropyl carbonate (MPC), ethylpropyl carbonate (EPC), and ethylmethyl carbonate (EMC).

The non-aqueous organic solvent according to the most specific embodiment may be a mixed solvent of dimethyl carbonate (DMC) and ethylmethyl carbonate (EMC).

The non-aqueous organic solvent may include ethylmethyl carbonate (EMC) and dimethyl carbonate (DMC) in a weight ratio of 0:100 to 50:50.

It may be more advantageous in terms of improving battery characteristics that the non-aqueous organic solvent includes dimethyl carbonate (DMC) in an amount of more than 50 wt%.

For example, the non-aqueous organic solvent may include ethylmethyl carbonate (EMC) and dimethyl carbonate (DMC) in a weight ratio of 0:100 to 40:60, 0:100 to 30:70, 10:90 to 40:60, or 10:90 to 30:70.

The non-aqueous organic solvent may further include an aromatic hydrocarbon-based solvent in addition to the carbonate-based solvent. In this case, the carbonate-based solvent and the aromatic hydrocarbon-based solvent may be mixed in a volume ratio of 1:1 to 30:1.

The aromatic hydrocarbon-based solvent may include an aromatic hydrocarbon-based compound represented by Chemical Formula 4.

[Chemical Formula 4]

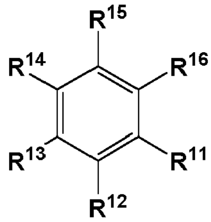

In Chemical Formula 4, $R^{11}$ to $R^{16}$ are the same as or different from each other and are selected from hydrogen, a halogen, a C1 to C10 alkyl group, a C1 to C10 haloalkyl group, and a combination thereof.

Specific examples of the aromatic hydrocarbon-based solvent may include benzene, fluorobenzene, 1,2-difluorobenzene, 1,3-difluorobenzene, 1,4-difluorobenzene, 1,2,3-trifluorobenzene, 1,2,4-trifluorobenzene, chlorobenzene, 1,2-dichlorobenzene, 1,3-dichlorobenzene, 1,4-dichlorobenzene, 1,2,3-trichlorobenzene, 1,2,4-trichlorobenzene, iodobenzene, 1,2-diiodobenzene, 1,3-diiodobenzene, 1,4-diiodobenzene, 1,2,3-triiodobenzene, 1,2,4-triiodobenzene, toluene, fluorotoluene, 2,3-difluorotoluene, 2,4-difluorotoluene, 2,5-difluorotoluene, 2,3,4-trifluorotoluene, 2,3,5-trifluorotoluene, chlorotoluene, 2,3-dichlorotoluene, 2,4-dichlorotoluene, 2,5-dichlorotoluene, 2,3,4-trichlorotoluene, 2,3,5-trichlorotoluene, iodotoluene, 2,3-diiodotoluene, 2,4-diiodotoluene, 2,5-diiodotoluene, 2,3,4-triiodotoluene, 2,3,5-triiodotoluene, xylene, or a combination thereof.

The lithium salt dissolves in a non-aqueous organic solvent and acts as a source of lithium ions in the battery to enable basic operation of the rechargeable lithium battery and promotes movement of lithium ions between the positive electrode and the negative electrode. Representative examples of such lithium salts may include $LiPF_6$, $LiBF_4$, lithium difluoro(oxalate)borate (LiDFOB), $LiPO_2F_2$, $LiSbF_6$, $LiAsF_6$, $Li(FSO_2)_2N$ (lithium bis(fluorosulfonyl)imide: LiFSI), $LiC_4F_9SO_3$, $LiClO_4$, $LiAlO_2$, $LiAlCl_4$, $LiN(C_xF_{2x+1}SO_2)(C_yF_{2y+1}SO_2)$, where x and y are integers selected from 1 to 20, LiCl, LiI, or $LiB(C_2O_4)_2$ (lithium bis(oxalato) borate: LiBOB).

A concentration of the lithium salt may be used within the range of 0.1 M to 2.0 M. When the lithium salt is included at the above concentration range, an electrolyte may have excellent performance and lithium ion mobility due to optimal electrolyte conductivity and viscosity.

At least one of $R^1$ to $R^4$ in Chemical Formula 1 may be a C1 to C20 alkyl group optionally substituted with an electron withdrawing group, a C6 to C20 aryl group optionally substituted with an electron withdrawing group, or a C2 to C30 heterocyclic group optionally substituted with an electron withdrawing group.

The electron withdrawing group may be at least one selected from a halogen, an isocyanate group (-NCO), an isothiocyanate group (-NCS), a cyanate group (-OCN), a thiocyanate group (-SCN), a cyano group (-CN), an isocyano group (-NC), a -N=C=N-group, a -N=S=N- group, a nitro group ($NO_2$), a trifluoromethane ($CF_3$) group, a pentafluoroethane ($C_2F_5$) group, a trifluoromethane sulfonyl ($SO_2CF_3$) group, a pentafluoroethane sulfonyl ($SO_2C_2F_5$) group, a trifluoromethane sulfonate ($SO_3CF_3$) group, a pentafluoroethane sulfonate ($SO_3C_2F_5$) group, a pentafluorophenyl ($C_6F_5$) group, an acetyl ($COCH_3$) group, an ethyl ketone ($COC_2H_5$) group, a propyl ketone ($COC_3H_7$) group, a butyl ketone ($COC_4H_9$) group, a pentyl ketone ($COC_5H_{11}$) group, a hexyl ketone ($COC_6H_{13}$) group, an ethanoate ($CO_2CH_3$) group, a propanoate ($CO_2C_2H_5$) group, a butaneoate ($CO_2C_3H_7$) group, a pentanoate ($CO_2C_4H_9$) group, and a hexanoate ($CO_2C_5H_{11}$) group.

At least one of $R^2$ and $R^3$ in Chemical Formula 1 may be hydrogen.

$R^2$ and $R^3$ in Chemical Formula 1 may each be hydrogen.

The compound represented by Chemical Formula 1 may be selected from the compounds of Group 1.

[Group 1]

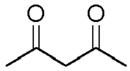
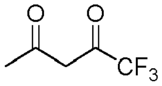
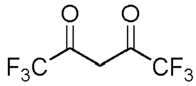
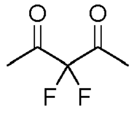
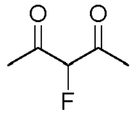

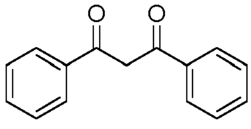
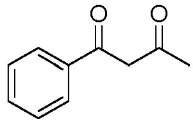
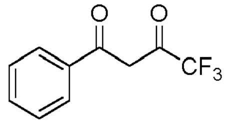
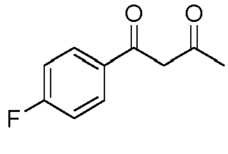

The additive may be included in an amount of 0.01 to 5.0 parts by weight based on 100 parts by weight of the electrolyte solution for a rechargeable lithium battery.

For example, the additive may be included in an amount of 0.01 to 3.0 parts by weight based on 100 parts by weight of the electrolyte solution for a rechargeable lithium battery.

For example, the additive may be included in an amount of 0.1 to 3.0 parts by weight, 0.3 to 3.0 parts by weight, 0.5 to 3.0 parts by weight, or 0.5 to 2.0 parts by weight based on 100 parts by weight of the electrolyte solution for a rechargeable lithium battery.

When the content range of the additive is as described above, it is possible to implement a rechargeable lithium battery having improved cycle-life characteristics and output characteristics by preventing an increase in resistance at high temperatures.

On the other hand, the electrolyte solution may further include at least one of other additives selected from vinylene carbonate (VC), fluoroethylene carbonate (FEC), difluoroethylene carbonate, chloroethylene carbonate, dichloroethylene carbonate, bromoethylene carbonate, dibromoethylene carbonate, nitroethylene carbonate, cyanoethylene carbonate, vinylethylene carbonate (VEC), adiponitrile (AN), succinonitrile (SN), 1,3,6-hexane tricyanide (HTCN), propensultone (PST), propanesultone (PS), lithium tetrafluoroborate ($LiBF_4$), lithium difluorophosphate ($LiPO_2F_2$), and 2-fluorobiphenyl (2-FBP).

By further including the aforementioned other additives, the cycle-life may be further improved or gases generated from the positive electrode and the negative electrode may be effectively controlled during high-temperature storage.

The other additives may be included in an amount of 0.2 to 20 parts by weight, specifically 0.2 to 15 parts by weight, or for example, 0.2 to 10 parts by weight, based on 100 parts by weight of the electrolyte solution for a rechargeable lithium battery.

When the amount of other additives is as described above, the increase in film resistance may be minimized, thereby contributing to the improvement of battery performance.

The positive electrode includes a positive electrode current collector and a positive electrode active material layer formed on the positive electrode current collector, and the positive electrode active material layer includes a positive electrode active material.

The positive electrode active material may include cobalt-free lithium nickel manganese-based oxide represented by Chemical Formula 2.

[Chemical Formula 2] $Li_aNi_xMn_{1-x-y}A_yO_{2\pm b}X_c$

In Chemical Formula 2,

$$0.9 \leq a \leq 1.2,\ 0 \leq b \leq 0.1,\ 0 \leq c \leq 0.1,\ 0.5 \leq x \leq 0.95, \text{ and } 0 \leq y < 0.3,$$

A is at least one element selected from B, Na, Mg, Al, Ti, and Si, and
X is at least one element selected from S, F, P, and Cl.

Of course, one having a coating layer on the surface of the lithium composite oxide may be used, or a mixture of the lithium composite oxide and a compound having a coating layer may be used. The coating layer may include at least one coating element compound selected from an oxide of a coating element, a hydroxide of a coating element, an oxyhydroxide of a coating element, an oxycarbonate of a coating element, and a hydroxy carbonate of a coating element. The compound for the coating layer may be amorphous or crystalline. The coating element included in the coating layer may include Mg, Al, K, Na, Ca, Si, Ti, V, Sn, Ge, Ga, B, As, Zr, or a mixture thereof. The coating layer may be disposed in a method having no adverse influence on properties of a positive electrode active material by using these elements in the compound. For example, the method may include any coating method (e.g., spray coating, dipping, etc.), but is not illustrated in more detail since it is well-known to those skilled in the related field.

For example, x in Chemical Formula 2 may be $0.8 \leq x \leq 0.95$.

For example, the positive electrode active material may include $LiNi_{0.75}Mn_{0.25}O_2$, $LiNi_{0.80}Mn_{0.20}O_2$, $LiNi_{0.85}Mn_{0.15}O_2$, $LiNi_{0.90}Mn_{0.10}O_2$, or $LiNi_{0.95}Mn_{0.05}O_2$, and a solid solution thereof.

The positive electrode active material may be included in an amount of 90 wt% to 98 wt% based on a total weight of the positive electrode active material layer.

In an embodiment, the positive electrode active material layer may further include a binder and a conductive material. Herein, each content of the binder and conductive material may be 1 wt% to 5 wt% based on a total weight of the positive electrode active material layer.

The binder improves binding properties of positive electrode active material particles with one another and with

a positive electrode current collector. Examples thereof may be polyvinyl alcohol, carboxylmethyl cellulose, hydroxypropyl cellulose, diacetyl cellulose, polyvinylchloride, carboxylated polyvinylchloride, polyvinylfluoride, an ethylene oxide-containing polymer, polyvinylpyrrolidone, polyurethane, polytetrafluoroethylene, polyvinylidene fluoride, polyethylene, polypropylene, a styrene-butadiene rubber, an acrylated styrene-butadiene rubber, an epoxy resin, nylon, and the like, but is not limited thereto.

The positive electrode current collector may be Al, but is not limited thereto.

The negative electrode includes a negative electrode current collector and a negative electrode active material layer including the negative electrode active material formed on the negative electrode current collector.

The negative electrode active material includes a material that reversibly intercalates/deintercalates lithium ions, a lithium metal, a lithium metal alloy, a material capable of doping/dedoping lithium, or a transition metal oxide.

The material that reversibly intercalates/deintercalates lithium ions includes carbon materials. The carbon material may be any generally-used carbon-based negative electrode active material in a rechargeable lithium battery. Examples of the carbon material include crystalline carbon, amorphous carbon, or a combination thereof. The crystalline carbon may be non-shaped, or sheet, flake, spherical, or fiber shaped natural graphite or artificial graphite. The amorphous carbon may be a soft carbon, a hard carbon, a mesophase pitch carbonized product, calcined coke, and the like.

The lithium metal alloy be an alloy of lithium and a metal selected from Na, K, Rb, Cs, Fr, Be, Mg, Ca, Sr, Si, Sb, Pb, In, Zn, Ba, Ra, Ge, Al, and Sn.

The material capable of doping and dedoping lithium may include Si, $SiO_x$ ($0 < x < 2$), a Si-Q alloy (wherein Q is selected from an alkali metal, an alkaline-earth metal, a Group 13 element, a Group 14 element excluding Si, a Group 15 element, a Group 16 element, a transition metal, a rare earth element, and a combination thereof), Sn, $SnO_2$, Sn-$R^{11}$ (wherein $R^{11}$ is an alkali metal, an alkaline-earth metal, a Group 13 element, a Group 14 element excluding Sn, a Group 15 element, a Group 16 element, a transition element, a rare earth element, or a combination thereof), and the like. At least one of them may be mixed with $SiO_2$.

The elements Q and $R^{11}$ may be selected from Mg, Ca, Sr, Ba, Ra, Sc, Y, Ti, Zr, Hf, Rf, V, Nb, Ta, Db, Cr, Mo, W, Sg, Tc, Re, Bh, Fe, Pb, Ru, Os, Hs, Rh, Ir, Pd, Pt, Cu, Ag, Au, Zn, Cd, B, Al, Ga, Sn, In, Tl, Ge, P, As, Sb, Bi, S, Se, Te, Po, and a combination thereof.

The transition metal oxide may be a vanadium oxide, a lithium vanadium oxide, and the like.

In a specific embodiment, the negative electrode active material may include at least one of graphite and a Si composite.

The Si composite may include a core including Si-based particles and an amorphous carbon coating layer, and the Si-based particles may include at least one of silicon particles, a Si-C composite, $SiO_x$ ($0 < x \leq 2$), and a Si alloy.

For example, a void may be included in the center portion of the core including the Si-based particles, the radius of the center portion may correspond to about 30% to about 50% of the radius of Si composite, an average particle diameter of the Si composite may be about 5 $\mu$m to about 20 $\mu$m, and an average particle diameter of the Si-based particles may be about 10 nm to about 200 nm.

In the present specification, an average particle diameter (D50) may be a particle size at a volume ratio of 50% in a cumulative size-distribution curve.

When the average particle diameter of the Si-based particles is within the above range, volume expansion occurring during charging and discharging may be suppressed, and disconnection of a conductive path due to particle crushing during charging and discharging may be prevented.

The core including the Si-based particles may further include amorphous carbon, and at this time, the center portion does not include amorphous carbon, and the amorphous carbon may exist only in the surface portion of the Si composite.

At this time, the surface portion means a region from the outermost surface of the central portion to the outermost surface of the Si composite.

In addition, the Si-based particles are substantially uniformly included throughout the Si composite, and may be present at a substantially uniform concentration in the center and surface portions.

The amorphous carbon may be soft carbon, hard carbon, a mesophase pitch carbonized product, calcined coke, or a combination thereof.

For example, the Si-C composite may include silicon particles and crystalline carbon.

The silicon particles may be included in an amount of about 1 wt% to about 60 wt%, for example, about 3 wt% to about 60 wt% based on the total weight of the Si-C composite.

The crystalline carbon may be, for example, graphite, and specifically, natural graphite, artificial graphite, or a combination thereof.

An average particle diameter of the crystalline carbon may be about 5 $\mu$m to about 30 $\mu$m.

When the negative electrode active material includes graphite and Si composite together, the graphite and Si composite may be included as a mixture, and in this case, the graphite and Si composite may be included in a weight ratio of about 99:1 to about 50:50.

More specifically, the graphite and Si composite may be included in a weight ratio of about 97:3 to about 80:20, or about 95:5 to about 80:20.

The amorphous carbon precursor may be coal-based pitch, mesophase pitch, petroleum-based pitch, coal-based oil, petroleum-based heavy oil, or a polymer resin such as a phenol resin, a furan resin, or a polyimide resin.

In the negative electrode active material layer, the negative electrode active material may be included in an amount of about 95 wt% to about 99 wt% based on the total weight of the negative electrode active material layer.

In an embodiment, the negative electrode active material layer may include a binder, and optionally a conductive material. In the negative electrode active material layer, the amount of the binder may be about 1 wt% to about 5 wt% based on a total weight of the negative electrode active material layer. When it further includes the conductive material, it may include about 90 wt% to about 98 wt% of the negative electrode active material, about 1 wt% to about 5 wt% of the binder, and about 1 wt% to about 5 wt% of the conductive material.

The binder improves binding properties of negative electrode active material particles with one another and with a negative electrode current collector. The binder may be a non-water-soluble binder, a water-soluble binder, or a combination thereof.

The non-water-soluble binder may be polyvinylchloride, carboxylated polyvinylchloride, polyvinylfluoride, polyurethane, polytetrafluoroethylene, polyvinylidene fluoride, polyethylene, polypropylene, polyamideimide, polyimide, or a combination thereof.

The water-soluble binder may be a rubber-based binder or a polymer resin binder. The rubber-based binder may be selected from a styrene-butadiene rubber, an acrylated styrene-butadiene rubber (SBR), an acrylonitrile-butadiene rubber, an acrylic rubber, a butyl rubber, a fluorine rubber, and a combination thereof. The polymer resin binder may be selected from polytetrafluoroethylene, an ethylenepropylene copolymer, polyethyleneoxide, polyvinylpyrrolidone, polyepichlorohydrine, polyphosphazene, polyacrylonitrile, polystyrene, ethylenepropylenedienecopolymer, polyvinylpyridine, chlorosulfonatedpolyethylene, latex, a polyester resin, an acrylic resin, a phenolic resin, an epoxy resin, polyvinyl alcohol, and a combination thereof.

When the water-soluble binder is used as the negative electrode binder, a cellulose-based compound may be further used to provide viscosity as a thickener. The cellulose-based compound includes one or more of carboxylmethyl cellulose, hydroxypropylmethyl cellulose, methyl cellulose, and an alkali metal salt thereof. The alkali metal may be Na, K, or Li. Such a thickener may be included in an amount of about 0.1 parts by weight to about 3 parts by weight based on 100 parts by weight of the negative electrode active material.

The conductive material is used to impart conductivity to the electrode, and in the configured battery, any material that does not cause chemical change and is electronically conductive may be used. For example, the conductive material may be a carbon-based material such as natural graphite, artificial graphite, carbon black, acetylene black, ketjen black, or a carbon fiber; a metal-based material such as a metal powder or a metal fiber including copper, nickel, aluminum, or silver; a conductive polymer such as a polyphenylene derivative; or a mixture thereof.

The negative electrode current collector may be selected from a copper foil, a nickel foil, a stainless steel foil, a titanium foil, a nickel foam, a copper foam, a polymer substrate coated with a conductive metal, and a combination thereof.

Depending on the type of rechargeable lithium battery, a separator may be present between the positive electrode and the negative electrode. Such a separator material may include polyethylene, polypropylene, polyvinylidene fluoride, or multi-layers thereof such as a polyethylene/polypropylene double-layered separator, a polyethylene/polypropylene/polyethylene triple-layered separator, or a polypropylene/polyethylene/polypropylene triple-layered separator.

The rechargeable lithium battery may have a charging upper limit voltage of greater than or equal to about 4.35 V. For example, the charging upper limit voltage may be about 4.35 V to about 4.55 V.

Hereinafter, examples of the present invention and comparative examples are described. These examples, however, are not in any sense to be interpreted as limiting the scope of the invention.

## Manufacture of Rechargeable Lithium Battery Cells

### Example 1

$LiNi_{0.75}Mn_{0.23}Al_{0.02}O_2$ as a positive electrode active material, polyvinylidene fluoride as a binder, and acetylene black as a conductive material were mixed in a weight ratio of 96:3:1 and then, dispersed in N-methyl pyrrolidone, preparing positive electrode active material slurry.

The positive electrode active material slurry was coated on a 15 μm-thick Al foil, dried at 100 °C, and pressed, manufacturing a positive electrode.

Negative electrode active material slurry was prepared by using a mixture of artificial graphite and an Si-C composite in a weight ratio of 93:7 as a negative electrode active material, mixing the negative electrode active material, a styrene-butadiene rubber binder, and carboxylmethyl cellulose in a weight ratio of 98:1:1, and dispersing the obtained

mixture in distilled water.

The Si composite had a core including artificial graphite and silicon particles and coated with coal pitch on the surface.

The negative electrode active material slurry was coated on a 10μm-thick Cu foil, dried at 100 °C, and pressed, manufacturing a negative electrode.

The manufactured positive and negative electrodes were assembled with a 10μm-thick polyethylene separator to manufacture an electrode assembly, and an electrolyte solution was injected thereinto, manufacturing a rechargeable lithium battery cell.

The composition of the electrolyte solution is as follows.

(Composition of Electrolyte Solution)

Lithium salt: 1.5 M $LiPF_6$
Non-aqueous organic solvent: ethylmethyl carbonate : dimethyl carbonate (EMC:DMC = weight ratio of 20:80)
Additive: 0.25 parts by weight of compound represented by Chemical Formula 1-1/ 10 parts by weight of fluoroethylene carbonate (FEC) / 0.5 parts by weight of succinonitrile (SN)

[Chemical Formula 1-1]

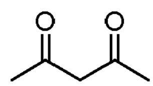

(in the composition of the electrolyte solution, "parts by weight" means a relative weight of an additive based on 100 weights of a total weight of an electrolyte solution excluding the additive (lithium salt + non-aqueous organic solvent).)

## Example 2

A rechargeable lithium battery cell was manufactured in the same manner as Example 1 except that the content of the compound represented by Chemical Formula 1-1 in the composition of the electrolyte solution was changed into 0.5 parts by weight.

## Example 3

A rechargeable lithium battery cell was manufactured in the same manner as Example 1 except that dimethyl carbonate (DMC) alone was used as the non-aqueous organic solvent in the composition of the electrolyte solution.

## Example 4

A rechargeable lithium battery cell was manufactured in the same manner as Example 1 except that the content of the compound represented by Chemical Formula 1-1 in the composition of the electrolyte solution was changed into 0.5 parts by weight, and the dimethyl carbonate (DMC) alone was used as the non-aqueous organic solvent.

## Example 5

A rechargeable lithium battery cell was manufactured in the same manner as Example 1 except that 0.5 parts by weight of the compound represented by Chemical Formula 1-2 was used instead of the compound represented by Chemical Formula 1-1 in the composition of the electrolyte solution.

[Chemical Formula 1-2]

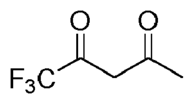

**Example 6**

A rechargeable lithium battery cell was manufactured in the same manner as Example 1 except that 0.5 parts by weight of the compound represented by Chemical Formula 1-3 was used instead of the compound represented by Chemical Formula 1-1 in the composition of the electrolyte solution.

[Chemical Formula 1-3]

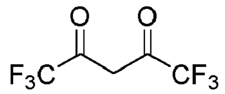

**Comparative Example 1**

A rechargeable lithium battery cell was manufactured in the same manner as Example 1 except that the compound represented by Chemical Formula 1-1 was not used in the composition of the electrolyte solution.

**Comparative Example 2**

A rechargeable lithium battery cell was manufactured in the same manner as Example 1 except that ethylene carbonate : ethylmethyl carbonate : dimethyl carbonate (in a weight ratio of EC:EMC:DMC=20:10:70) were used as the non-aqueous organic solvent in the composition of the electrolyte solution.

**Evaluation 1: Evaluation of Room-Temperature Charge/discharge Cycle Characteristics**

The rechargeable lithium battery cells according to Examples 1 to 6 and Comparative Examples 1 to 2 were charged and discharged under the following conditions and evaluated with respect to cycle characteristics, and the results are shown in Table 1.

After performing 200 cycles of the charge and discharge under the 0.33 C charge (CC/CV, 4.45 V, 0.025 C cut-off) /1.0 C discharge (CC, 2.5 V cut-off) conditions at 25 °C, the cells were measured with respect to changes in capacity retention rate and direct current internal resistance (DC-IR) change.

DC-IR was calculated according to Equations 1 and 2 based on a voltage changed by discharging the cells, while applying a current of SOC 50% for 30 seconds, and the results are shown in Table 1.

[Equation 1]

$$\text{Capacity retention rate} = (\text{capacity after 200 cycles} / \text{capacity after 1 cycle}) * 100$$

[Equation 2]

$$\text{DC-IR change} = (\text{DC-IR after 200 cycles})/(\text{DC-IR after 1 cycle}) * 100$$

(Table 1)

| | Capacity retention rate (@25 °C, 200 Cycles) (%) | DC-IR after 1 cycle @25 °C (mΩ) | DC-IR after 200cycles @25 °C (mΩ) | DC-IR change @25 °C (%) |
|---|---|---|---|---|
| Example 1 | 91.7 | 43.4 | 84.2 | 194% |
| Example 2 | 92.2 | 43.8 | 83.8 | 191% |
| Example 3 | 92.4 | 45.5 | 85.2 | 187% |
| Example 4 | 93.2 | 46.1 | 84.8 | 184% |

(continued)

| | Capacity retention rate (@25 °C, 200 Cycles) (%) | DC-IR after 1 cycle @25 °C (mΩ) | DC-IR after 200cycles @25 °C (mΩ) | DC-IR change @25 °C (%) |
|---|---|---|---|---|
| Example 5 | 95.1 | 44.7 | 81.5 | 182% |
| Example 6 | 93.0 | 45.1 | 82.6 | 183% |
| Comparative Example 1 | 89.9 | 42.9 | 85.7 | 200% |
| Comparative Example 2 | 89.8 | 42.8 | 83.4 | 195% |

Referring to Table 1, when the additive according to the present invention was used, room-temperature cycle-life characteristics were improved.

**Evaluation 2: Evaluation of High-Temperature (45 °C) Cycle-life characteristics**

The rechargeable lithium battery cells according to Examples 1 to 6 and Comparative Examples 1 to 2 were 200 cycles charged and discharged under 0.33 C charge (CC/CV, 4.45 V, 0.025 C cut-off) / 1.0 C discharge (CC, 2.5 V cut-off) conditions at 45 °C and then, evaluated with respect to changes in capacity retention rate and direct current internal resistance (DC-IR) change.

DC-IR was calculated according to Equations 1 and 2 based on a voltage changed by discharging the cells, while applying a current of SOC 50 C for 30 seconds, and the results are shown in Table 2.

(Table 2)

| | Capacity retention rate (@ 45 °C, 200 Cycles) (%) | DC-IR after 1 cycle @ 45 °C (mΩ) | DC-IR after 200 cycles @ 45 °C (mΩ) | DC-IR change @ 45 °C (%) |
|---|---|---|---|---|
| Example 1 | 89.6 | 50 | 106 | 212 |
| Example 2 | 90.1 | 50.4 | 105.6 | 210 |
| Example 3 | 90.3 | 52.1 | 107 | 205 |
| Example 4 | 91.1 | 52.7 | 106.6 | 202 |
| Example 5 | 95.1 | 44.7 | 81.5 | 182 |
| Example 6 | 93.0 | 45.1 | 82.6 | 183 |
| Comparative Example 1 | 87.8 | 49.5 | 107.5 | 217 |
| Comparative Example 2 | 87.7 | 49.4 | 105.2 | 213 |

Referring to Table 2, when the additive according to the present invention was used, high-temperature cycle-life characteristics were improved.

**Evaluation 3: Evaluation of High-Temperature Storage Characteristics (Capacity Retention Rate/Capacity Recovery Rate/DC-IR)**

The rechargeable lithium battery cells according to Examples 1 to 6 and Comparative Examples 1 to 2 were once charged and discharged at 0.33 C and then, measured with respect to charge and discharge capacity (before high-temperature storage).

In addition, the rechargeable lithium battery cells according to Examples 1 to 6 and Comparative Examples 1 to 2 were charged to SOC100% (a charge state to 100% of charge capacity based on 100% of total battery charge capacity) and stored at 60 °C for 30 days, and discharged to 3.0 V at 0.33 C under a constant current condition and

then, measured with respect to initial discharge capacity.

The cells were recharged to 4.3 V at 0.33 C under a constant current condition and cut off at 0.02 C under a constant voltage condition and discharged to 3.0 V at 0.33 C under a constant current condition and then, twice measured with respect to discharge capacity. A ratio of the first discharge capacity to the initial discharge capacity was provided as capacity retention rate, and the second discharge capacity was provided as capacity recovery rate.

The rechargeable lithium battery cells according to Examples 1 to 6 and Comparative Examples 1 to 2 were measured with respect to △V/△I (voltage change / current change) to obtain initial DC internal resistance (DC-IR), fully charged (SOC 100%) to secure an internal maximum energy state, stored at a high temperature of 60 °C for 30 days, and then, measured again with respect to DC internal resistance, which were used to calculate a DCIR increase rate (%) according to Equation 3, and the results are shown in Table 3.

[Equation 3]

$$\text{DCIR increase rate} = (\text{DCIR after 30 days} / \text{Initial DCIR}) * 100$$

(Table 3)

| | Capacity retention rate (@60 °C, 30D) (%) | Capacity recovery rate (@60 °C, 30D) (%) | Initial DCIR (@25 °C, mΩ) | DC-IR @60 °C , 30D (mΩ) | DC-IRincrease rate (%) |
|---|---|---|---|---|---|
| Example 1 | 90.8 | 94.2 | 43.4 | 96 | 221 |
| Example 2 | 91.3 | 94.7 | 43.8 | 95.6 | 218 |
| Example 3 | 91.5 | 94.9 | 45.5 | 97 | 213 |
| Example 4 | 92.3 | 95.7 | 46.1 | 96.6 | 210 |
| Example 5 | 96.3 | 97.6 | 44.7 | 93.3 | 209 |
| Example 6 | 94.2 | 95.5 | 45.1 | 94.4 | 209 |
| Comparative Example 1 | 89 | 92.4 | 42.9 | 97.5 | 227 |
| Comparative Example 2 | 88.9 | 92.3 | 42.8 | 95.2 | 222 |

Referring to Table 3, the rechargeable lithium battery cells according to Examples 1 to 6 exhibited improved capacity retention rate and capacity recovery rate when stored at a high temperature and were suppressed from DC-IR increase rate, when compared to the cells according to Comparative Examples 1 to 2.

## Claims

**1.** A rechargeable lithium battery (100), comprising

an electrolyte solution including a non-aqueous organic solvent, a lithium salt, and an additive;
a positive electrode (114) including a positive electrode active material; and
a negative electrode (112) including a negative electrode active material,
wherein the non-aqueous organic solvent includes ethylene carbonate in an amount of less than 5 wt%,
the additive includes a compound represented by Chemical Formula 1, and
the positive electrode active material is represented by Chemical Formula 2:

[Chemical Formula 1]

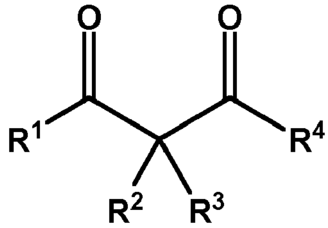

wherein, in Chemical Formula 1,
$R^1$ to $R^4$ are each independently hydrogen, a halogen, a cyano group, a substituted or unsubstituted C1 to C20 alkyl group, a substituted or unsubstituted C6 to C20 aryl group, or a substituted or unsubstituted C2 to C30 heterocyclic group;

[Chemical Formula 2] $Li_aNi_xMn_{1-x-y}A_yO_{2\pm b}X_c$

wherein, in Chemical Formula 2,

$$0.9 \leq a \leq 1.2,\ 0 \leq b \leq 0.1,\ 0 \leq c \leq 0.1,\ 0.5 \leq x \leq 0.95, \text{ and } 0 \leq y < 0.3,$$

A is at least one element selected from B, Na, Mg, Al, Ti, and Si, and
X is at least one element selected from S, F, P, and Cl.

**2.** The rechargeable lithium battery of claim 1, wherein
the non-aqueous organic solvent is composed of only chain carbonate.

**3.** The rechargeable lithium battery of claim 2, wherein
the chain carbonate is represented by Chemical Formula 3:

[Chemical Formula 3]

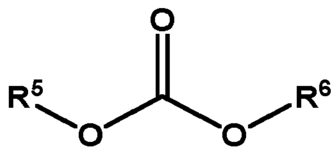

wherein, in Chemical Formula 3
$R^5$ and $R^6$ are each independently a substituted or unsubstituted C1 to C20 alkyl group.

**4.** The rechargeable lithium battery of claim 3, wherein
the non-aqueous organic solvent is a mixed solvent comprising at least two solvents of dimethyl carbonate (DMC), diethyl carbonate (DEC), dipropyl carbonate (DPC), methylpropyl carbonate (MPC), ethylpropyl carbonate (EPC), and ethylmethyl carbonate (EMC).

**5.** The rechargeable lithium battery of claim 1, wherein
the non-aqueous organic solvent includes ethylmethyl carbonate (EMC) and dimethyl carbonate (DMC) in a weight ratio of 0: 100 to 50: 50.

**6.** The rechargeable lithium battery of any one of the preceding claims, wherein
at least one selected from $R^1$ to $R^4$ in Chemical Formula 1 is an unsubstituted C1 to C20 alkyl group, a C1 to C20 alkyl group substituted with an electron withdrawing group, an unsubstituted C6 to C20 aryl group, a C6 to C20 aryl group substituted with an electron withdrawing group, an unsubstituted C2 to C30 heterocyclic group, or a C2 to C30 heterocyclic group substituted with an electron withdrawing group.

**7.** The rechargeable lithium battery of claim 6, wherein
the electron withdrawing group is at least one selected from a halogen, an isocyanate group (-NCO), an isothiocyanate group (-NCS), a cyanate group (-OCN), a thiocyanate group (-SCN), a cyano group (-CN), an isocyano group (-NC), a - N=C=N- group, a -N=S=N- group, a nitro group ($NO_2$), a trifluoromethane ($CF_3$) group, a pentafluoroethane ($C_2F_5$) group, a trifluoromethane sulfonyl ($SO_2CF_3$) group, a pentafluoroethane sulfonyl ($SO_2C_2F_5$) group, a trifluoromethane sulfonate ($SO_3CF_3$) group, a pentafluoroethane sulfonate ($SO_3C_2F_5$) group, a pentafluorophenyl ($C_6F_5$) group, an acetyl ($COCH_3$) group, an ethyl ketone ($COC_2H_5$) group, a propyl ketone ($COC_3H_7$) group, a butyl ketone ($COC_4H_9$) group, a pentyl ketone ($COC_5H_{11}$) group, a hexyl ketone ($COC_6H_{13}$) group, an ethanoate ($CO_2CH_3$) group, a propanoate ($CO_2C_2H_5$) group, a butaneoate ($CO_2C_3H_7$) group, a pentanoate ($CO_2C_4H_9$) group, and a hexanoate ($CO_2C_5H_{11}$) group.

**8.** The rechargeable lithium battery of any one of the preceding claims, wherein
at least one of $R^2$ and $R^3$ in Chemical Formula 1 is hydrogen.

**9.** The rechargeable lithium battery of claim 8, wherein
$R^2$ and $R^3$ of Chemical Formula 1 are each hydrogen.

**10.** The rechargeable lithium battery of claim 1, wherein
the compound represented by Chemical Formula 1 is selected from compounds of Group 1:

[Group 1]

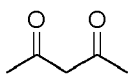 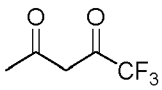 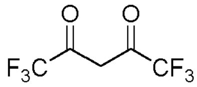 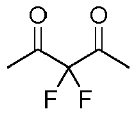 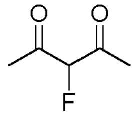

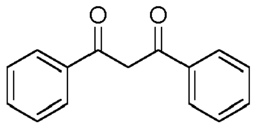 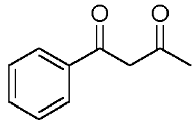 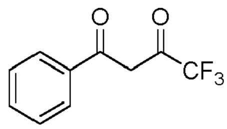 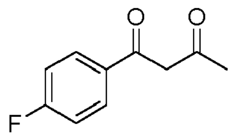 .

**11.** The rechargeable lithium battery of any one of the preceding claims, wherein
the compound represented by Chemical Formula 1 is included in an amount of 0.01 to 5.0 parts by weight based on 100 parts by weight of the electrolyte solution for a rechargeable lithium battery.

**12.** The rechargeable lithium battery of any one of the preceding claims, wherein
the electrolyte solution further includes at least one other additive selected from vinylene carbonate (VC), fluoroethylene carbonate (FEC), difluoroethylene carbonate, chloroethylene carbonate, dichloroethylene carbonate, bromoethylene carbonate, dibromoethylene carbonate, nitroethylene carbonate, cyanoethylene Carbonate, vinylethylene carbonate (VEC), adiponitrile (AN), succinonitrile (SN), 1,3,6-hexanetricyanide (HTCN), propensultone (PST), propanesultone (PS), lithium tetrafluoroborate ($LiBF_4$), lithium difluorophosphate ($LiPO_2F_2$), and 2-fluorobiphenyl (2-FBP).

**13.** The rechargeable lithium battery of any one of the preceding claims, wherein
x in Chemical Formula 2 is $0.8 \leq x \leq 0.95$.

**14.** The rechargeable lithium battery of claim 12, wherein
the positive electrode active material includes $LiNi_{0.75}Mn_{0.25}O_2$, $LiNi_{0.80}Mn_{0.20}O_2$, $LiNi_{0.85}Mn_{0.15}O_2$, $LiNi_{0.90}Mn_{0.10}O_2$, or $LiNi_{0.95}Mn_{0.05}O_2$, and a solid solution thereof.

**15.** The rechargeable lithium battery of any one of the preceding claims, wherein
the negative electrode active material includes at least one of graphite and a Si composite.

【FIG. 1】

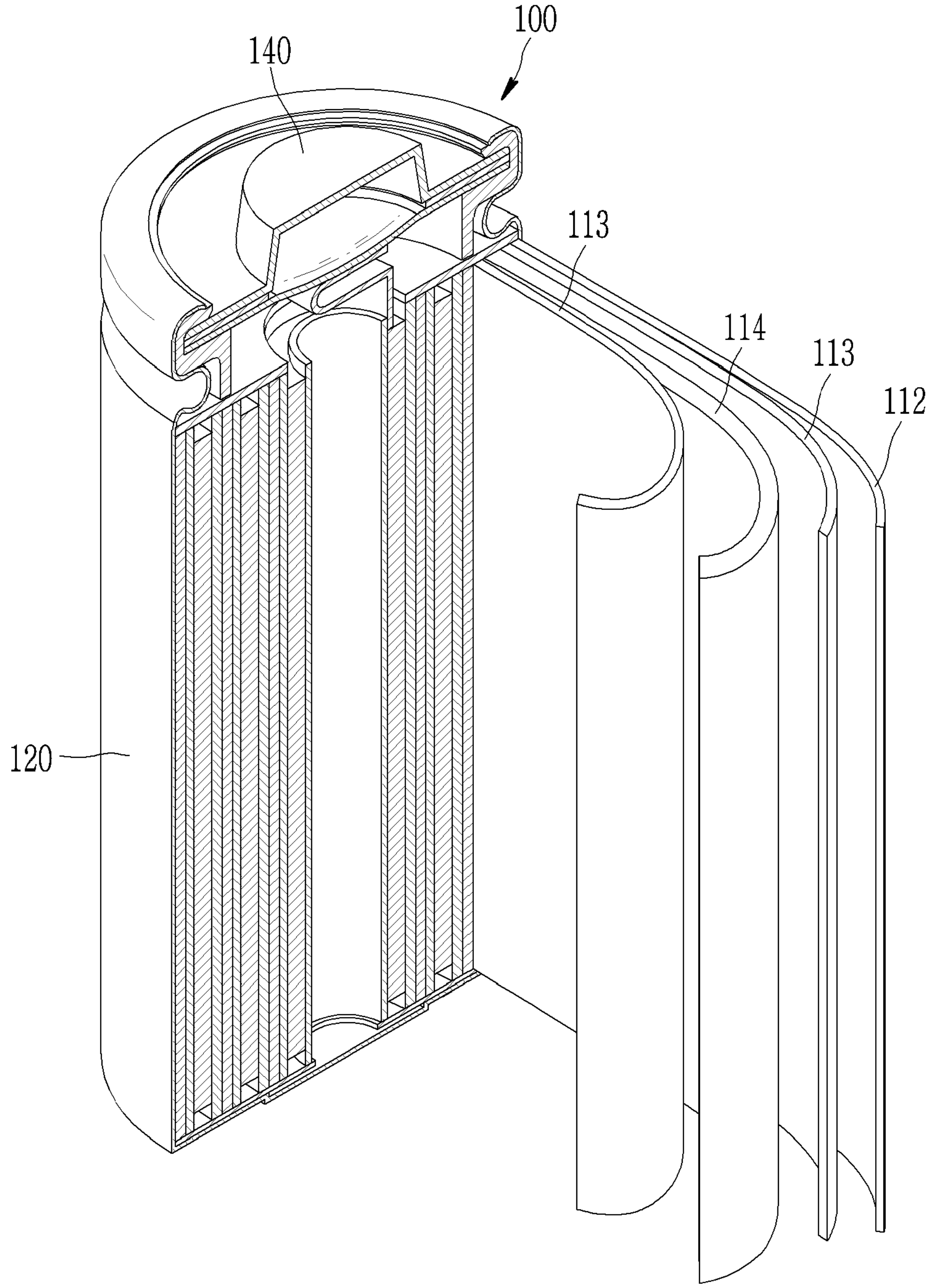

Europäisches Patentamt
European Patent Office
Office européen des brevets

# EUROPEAN SEARCH REPORT

Application Number
EP 23 19 5155

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 111 342 130 A (DO FLUORIDE NEW ENERGY TECHNOLOGY CO., LTD.) 26 June 2020 (2020-06-26)<br>* abstract *<br>* paragraph [0006] - paragraph [0010]; compound 1 *<br>* examples 1-5, 7 *<br>* claims 1-4 *<br>----- | 1-15 | INV.<br>H01M10/0567<br>H01M10/0569<br>H01M10/0525<br>H01M4/525<br><br>ADD.<br>H01M4/36<br>H01M4/38<br>H01M4/587 |
| A | EP 2 037 468 A1 (OTSUKA CHEMICAL CO., LTD.) 18 March 2009 (2009-03-18)<br>* paragraph [0006] - paragraph [0013] *<br>* paragraph [0028] - paragraph [0031] *<br>* paragraph [0042] *<br>* example 10; table 1 *<br>* examples 15, 20; table 2 *<br>* claims 1, 4-7 *<br>----- | 1-15 | |
| A | US 2008/153006 A1 (PANAX ETEC CO., LTD.) 26 June 2008 (2008-06-26)<br>* paragraph [0009] - paragraph [0010] *<br>* paragraph [0012] *<br>* paragraph [0021] - paragraph [0023] *<br>* paragraph [0027] - paragraph [0029] *<br>* examples 1-5; table 1 *<br>* claims 1-5, 13, 14, 17 *<br>----- | 1-15 | TECHNICAL FIELDS SEARCHED (IPC)<br><br>H01M |
| A | CN 112 952 057 A (SOUNDON NEW ENERGY TECHNOLOGY CO., LTD.) 11 June 2021 (2021-06-11)<br>* abstract *<br>* comparative examples 1-3; examples 1-4 *<br>* claims 1, 3-7, 9 *<br>-----<br>-/-- | 1-15 | |

The present search report has been drawn up for all claims

2

| Place of search | Date of completion of the search | Examiner |
|---|---|---|
| Munich | 27 February 2024 | Masson, Jean-Pierre |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 19 5155

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 112 382 734 A (A123 SYSTEMS LLC; WANXIANG GROUP CO., LTD.) 19 February 2021 (2021-02-19) * abstract * * comparative examples 1, 2; examples 1-3 * * claim 1 * ----- | 1-15 | |
| A | KR 2020 0099900 A (LG CHEM, LTD.) 25 August 2020 (2020-08-25) * abstract * * comparative examples 1-3; examples 1-5 * * claims 1, 5-8 * ----- | 1-15 | |
| A | JP 2009 163971 A (PANASONIC CORP.) 23 July 2009 (2009-07-23) * abstract * * examples 1-14; table 1 * * examples 19-30; table 2 * * claims 1-6 * ----- | 1-15 | |
| | | | TECHNICAL FIELDS SEARCHED (IPC) |

The present search report has been drawn up for all claims

2

| Place of search | Date of completion of the search | Examiner |
|---|---|---|
| Munich | 27 February 2024 | Masson, Jean-Pierre |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 23 19 5155

This annex lists the patent family members relating to the patent documents cited in the above-mentioned European search report. The members are as contained in the European Patent Office EDP file on
The European Patent Office is in no way liable for these particulars which are merely given for the purpose of information.

27-02-2024

| Patent document cited in search report | Publication date | Patent family member(s) | Publication date |
|---|---|---|---|
| CN 111342130 A | 26-06-2020 | NONE | |
| EP 2037468 A1 | 18-03-2009 | CN 101479818 A | 08-07-2009 |
| | | EP 2037468 A1 | 18-03-2009 |
| | | JP 4802243 B2 | 26-10-2011 |
| | | JP WO2008001955 A1 | 03-12-2009 |
| | | KR 20090026203 A | 11-03-2009 |
| | | TW 200816243 A | 01-04-2008 |
| | | US 2009309060 A1 | 17-12-2009 |
| | | US 2012170172 A1 | 05-07-2012 |
| | | WO 2008001955 A1 | 03-01-2008 |
| US 2008153006 A1 | 26-06-2008 | CN 101207208 A | 25-06-2008 |
| | | KR 100767427 B1 | 17-10-2007 |
| | | US 2008153006 A1 | 26-06-2008 |
| | | US 2011045358 A1 | 24-02-2011 |
| CN 112952057 A | 11-06-2021 | NONE | |
| CN 112382734 A | 19-02-2021 | NONE | |
| KR 20200099900 A | 25-08-2020 | NONE | |
| JP 2009163971 A | 23-07-2009 | NONE | |

EPO FORM P0459

For more details about this annex : see Official Journal of the European Patent Office, No. 12/82